**Europäisches Patentamt**

(19) **European Patent Office**

**Office européen des brevets**

(11) Publication number : **0 418 266 B1**

(12) # EUROPEAN PATENT SPECIFICATION

(45) Date of publication of patent specification :
**11.11.92 Bulletin 92/46**

(51) Int. Cl.⁵ : **A61B 10/00, A46B 3/00**

(21) Application number : **89905819.2**

(22) Date of filing : **10.05.89**

(86) International application number :
**PCT/SE89/00258**

(87) International publication number :
**WO 89/10724 16.11.89 Gazette 89/27**

(54) **BRUSH AND METHOD IN THE MANUFACTURE THEREOF.**

(30) Priority : **10.05.88 SE 8801764**

(43) Date of publication of application :
**27.03.91 Bulletin 91/13**

(45) Publication of the grant of the patent :
**11.11.92 Bulletin 92/46**

(84) Designated Contracting States :
**AT BE CH DE FR GB IT LI LU NL SE**

(56) References cited :
**EP-A- 0 239 270
EP-A- 0 292 693
GB-A- 1 553 438
SE-B- 420 047**

(73) Proprietor : **STORMBY, Nils
Medscand AB P.O. Box 20047
S-200 74 Malmö 20 (SE)**

(72) Inventor : **HEDBERG, Tommy
Notariegränd 48
S-222 47 Lund (SE)**
Inventor : **CLAREN, Jan
Protokollgränden 38
S-222 47 Lund (SE)**

(74) Representative : **Ström, Tore et al
Ström & Gulliksson AB Studentgatan 1 P.O.
Box 4188
S-203 13 Malmö (SE)**

## Description

The invention relates to a brush, particularly for medical use, comprising a shank which is provided with bristles over a portion of the length thereof, preferably an end portion, and a method in manufacturing such a brush.

For medical sampling in body cavities and body passages there are used in addition to spatulas and cotton swabs, such swabs consisting of a shank with a cotton wad at one end thereof, also brushes because these, contrary to spatulas and cotton swabs, can be made of a material and with a stiffness and density which are adapted to the surface of the body at which the sampling is to be effected, in order to scrape sufficiently effectively on said surface as is required in order that the cells or surface fragments and/or secrete sought for shall be collected in and/or adhere to the brush, and the brush at the same time can be constructed such that it makes possible to perform a gentle and as far as possible painless sampling. A further advantage of the sampling brush is that the collected sample can be easily transferred from the brush by smearing on a microscope slide or by washing in a liquid without the collected cells being damaged or destroyed. Sampling brushes are used at present for sampling e.g. in uterus, cervix or urethra.

In present embodiments the sampling brush comprises a shank of twisted wire and bristles or bristle tufts are attached to the shank by being clamped between the twisted wires. Such sampling brushes are shown and described e.g. in US-A-4 227 537 (Suciu et al) and US-A-3 881 464 (Levene). The manufacture of such brushes is expensive and can be said to be too expensive for a one-way product as is concerned here, and the possibilities to make small brushes, i.e. brushes wherein the brush portion has a small diameter, and to make brushes with very thin bristles, i.e. softer brushes, are very limited. However, it is desired to provide small and soft brushes as well as to reduce the manufacturing costs of the brushes.

The purpose of the invention is to attain this particulary in case of brushes for medical sampling of the kind referred to above, e.g. for sampling in urethra, but the invention as to the broadest aspect thereof is not limited to brushes for sampling purposes because it is of importance also for brushes for other medical uses such as application of therapeutic substances or refrigerants on and in the human body, and also for brushes outside the medical field, e.g. brushes for application of cosmetics, particularly mascara, because brushes for this purpose should be kind to the sensitive skin on the eyelid and at the same time should be able to penetrate between the hairs of the eyelash.

The purpose mentioned above is attained by the brush of the invention having obtained the characteristics of claim 1, and for the manufacture of such a brush the invention also provides the method defined in claim 11.

The velour finishing technique per se is previously known in order to impart e.g. to surfaces the character of being covered with velvet but is applied here for a completely new purpose, viz. to provide a rough brushlike surface on a body.

Illustrative embodiments of the invention are shown in the accompanying drawings wherein

FIG 1 is a side view of a sampling brush having cylindrical shape,

FIG 2 is a half side view and a half axial crosssectional view of a sampling brush having a particular shape adapted to the sampling place,

FIG 3 is a side view of a brush of the invention having conical shape,

FIGS 4 and 5 are two side views perpendicular to each other of a further development of the brush of FIG 3 having a shape which is adapted to the sampling place,

FIG 6 is a side view of a sampling brush having the velour finished surface provided on a circular disk,

FIG 7 is a side view of a urethra brush,

FIG 8 is a side view of a brush provided on a conventional spatula,

FIG 9 is a side view of a conical brush having annular velour finished portions,

FIG 10 is a side view of a conical brush having a helically extending velour finished portion,

FIG 11 is a side view of a conical brush having axially extending velour finished portions,

FIG 12 is a perspective view of a number of brush shanks which have been produced by injection molding and are integral via a plateshaped ingot,

FIG 13 is a cross-sectional view of a guide rail for advancing the molding of FIG 12 along a process line,

FIG 14 is a diagrammatic side view of apparatus for applying glue to the shank ends in a station along the process line, and

FIG 15 is a diagrammatic side view of apparatus for applying fibers to the glue coated shank ends by electrostatic flocculation.

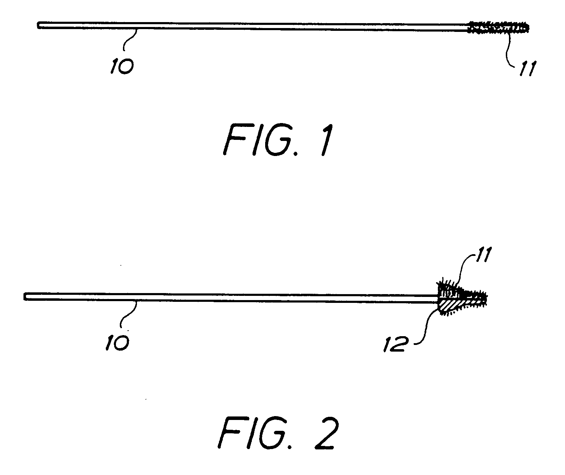

Referring to FIG 1 of the drawings a brush for medical use, in this case for medical sampling in the mouth of cervix, comprises a shank 10 which can consist of a solid or tubular plastics rod which is rigid or of such character that it can be bent plastically or elastically. One end portion of the shank is provided with bristles 11 applied by velour finishing this portion.

It is not necessary that the brush is made substantially cylindrical as in FIG 1. The brush may also be given another shape, e.g. conical shape or a shape which is adapted to the shape of the sampling place. An embodiment of the last-mentioned type is shown in FIG 2. A body 12 is shaped as the sampling place and is attached to or is integral with the shank 10, and the surface of this body then has been velour finished.

In a special embodiment of the brush in FIG 1 when it is intended for sampling in urethra the shank preferably is made very thin and is tapering conically from the brush end to the handle end in order that the shank shall be very resilient and easily shall adjust itself to urethra when it is inserted thereinto. Such a brush can have e.g. a minimum diameter of the shank at the brush end, which is 0.8 mm, and a maximum diameter at the handle end, which is 1.5 mm, the brush proper (the velour finished portion) having a length of 0.5 mm. A brush constructed in this way has been found to cause considerably less pain when used for sampling in urethra than conventional brushes having a shank of twisted wire and bristles attached thereto.

By applying the velour finishing technique there are provided unlimited possibilities of adjusting the shape of the brush to the specific sampling for which the brush is intended.

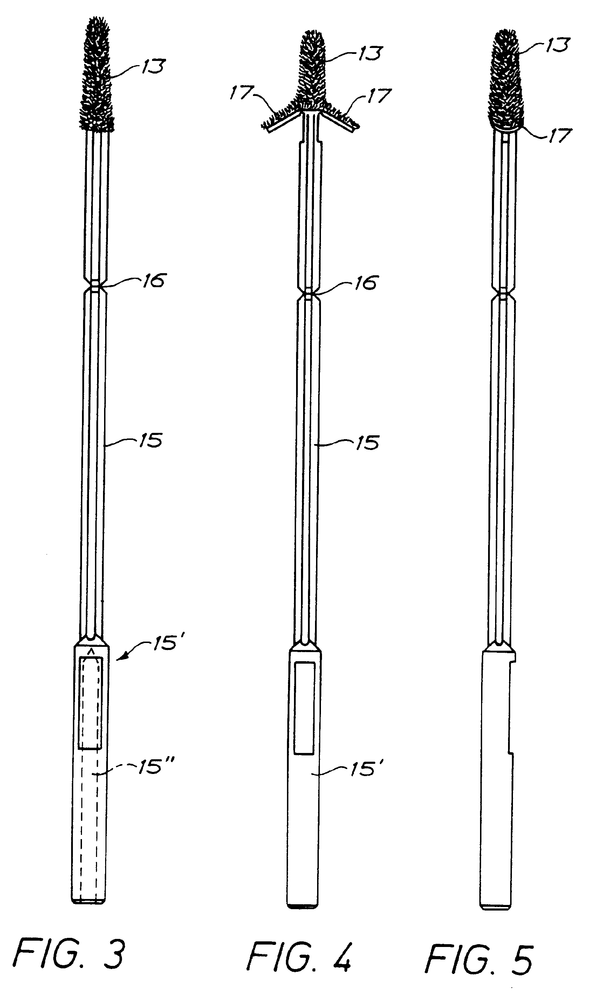

The brush in FIG 3 comprises a conical body 13 which is integral with a shank 15 preferabaly of cross-shaped cross-section so as to have sufficient rigidity, said shank forming a handle 15′ of cylindrical shape. Preferably, the shank is produced by injection molding of a suitable plastics material, e.g. polypropylene. For technical reasons from the manufacturing point of view an axial bottom hole 15" is provided in the handle to avoid concentration of material in the handle, which could cause impressions at the outside surface of the handle and disturb the cylindrical shape thereof. The conical body is velour finished at the conical surface thereof. The fibers of a brush for cytological sampling in the embodiment of the brush according to the invention, which is shown in FIG 3, typically can have a length of the order of 2 mm and a diameter of the order of 0.12 mm. Such a brush has been found to cause less tendency of bleeding at the sampling than such conventional brushes which are made with a shank of twisted wire having bristles attached thereto. A break-point 16 is arranged spaced from the conical body so that the end portion of the brush can be broken off when it is inserted into a test tube used for treating or transporting the sample that has been taken.

The further development of the brush of FIG 3 which is shown in FIGS 4 and 5 comprises two flaps 17 on the conical portion 13, said flaps being connected to said portion by means of integral hinges and are slightly curved so that they can be bent towards the shank and partly enclose the shank, e.g. when the brush after sampling is rolled against a microscope slide for smearing the sample thereon. The brush is shaped particularly for sampling in the cervical mouth.

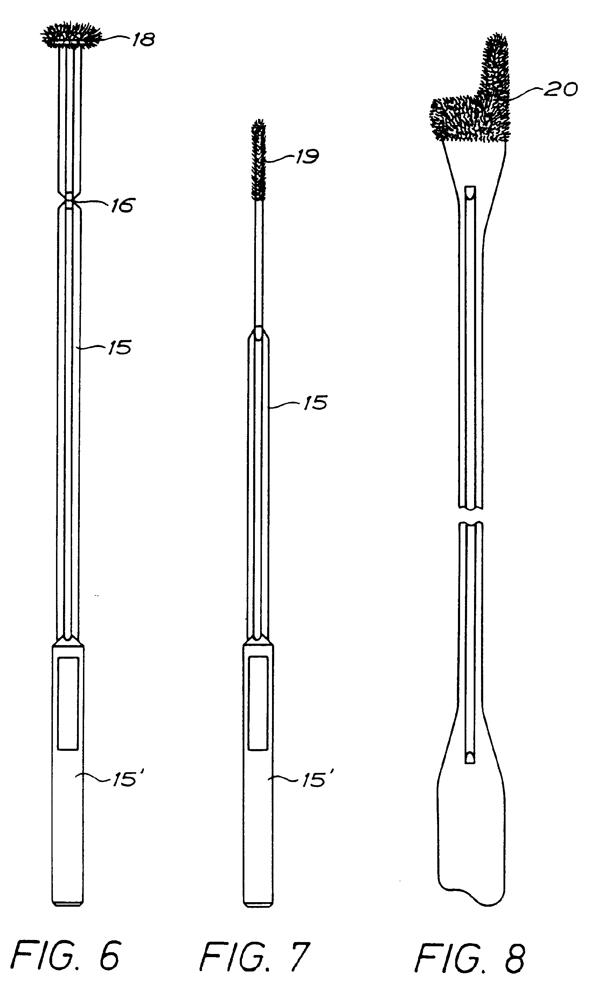

The brush in FIG 6 has a circular disk 18 which is velour finished and to which the shank is attached centrally and axially. This brush can be used for sampling but it is also very useful for applying medicaments at therapeutic treatment of local portions, such as warts, on the human body, or for applying a refrigerant (nitrogen) at cryotherapy.

The brush in FIG 7 has a velour finished portion 19 which has a substantially smaller cross dimension than the shank, and this brush is intended for use as a urethra brush.

As shown in FIG 8 a conventional spatula for sampling,in the cervical mouth can be provided with a velour finished portion 20.

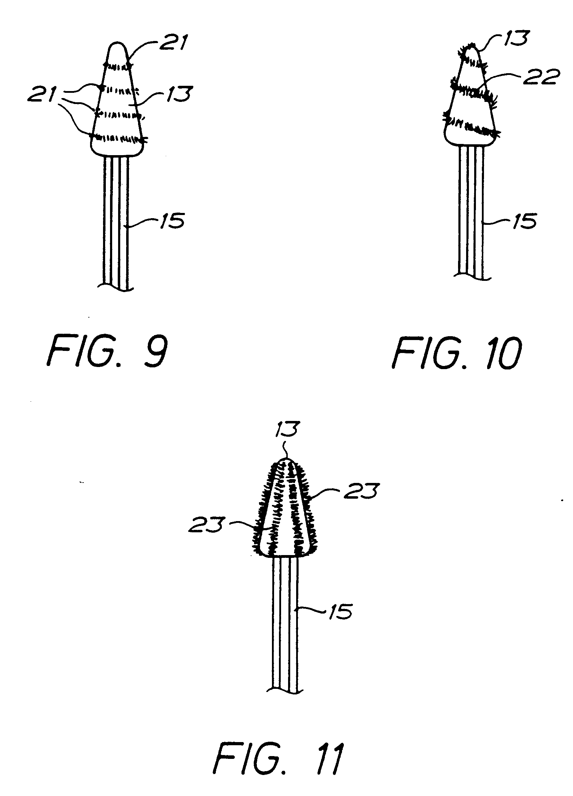

The velour finished surface can be divided into a number of axially mutually spaced annular velour finished portions 21 according to FIG 9, or can form a helically extending string 22, as shown in FIG 10. The velour finished surface can also include a number of peripherally mutually spaced axial portions 23 according to FIG 11.

The brush of the invention is kind to the tissues and at the same time is effective as far as scraping of secrete, surface fragments or cells is concerned. Moreover, the brush has a pronounced ability of retaining such material as has been collected by the sampling or such material as has been taken up by means of the brush so as to be applied to the human body, the brush at the same time willingly giving off such material when smeared on a microscope slide or the skin, respectively. The brush also has the ability to penetrate between the hairs of the eyelash when used for applying mascara, and it is not easily clogged when it is used for such application; the brush will stand fluffy also after having been used for a long time.

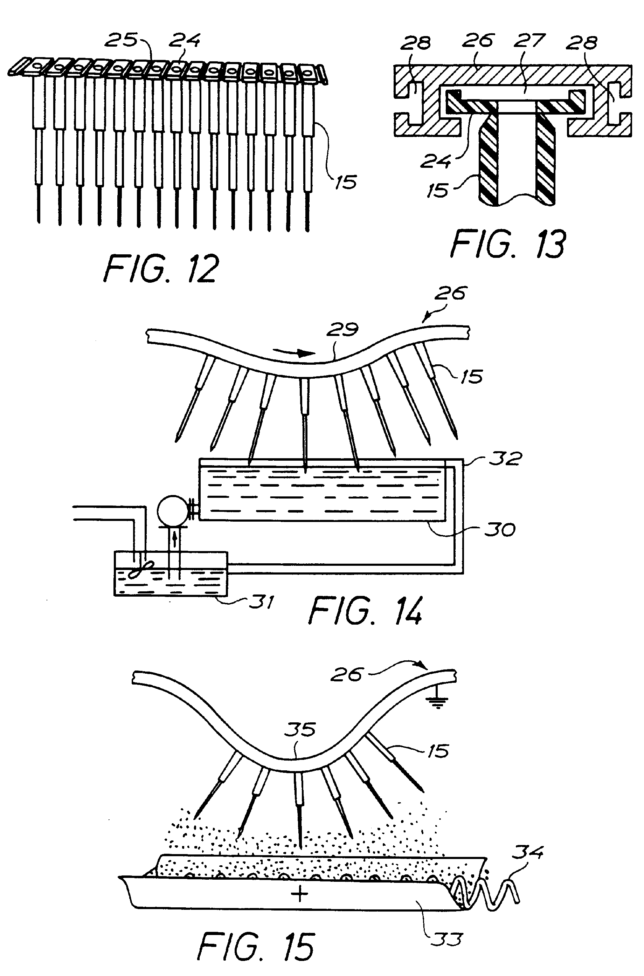

The method of the invention for manufacturing the brushes described will be explained in more detail with reference to FIGS 12 to 15.

The shanks 15 for several brushes can be injection molded in a single shot and in that case are integrally connected at an ingot 24 as shown in FIG 12, wherein the brush shanks are assumed to be of the embodiment of FIG 7. The ingot is plate-shaped and relatevely thin, and between adjacent shanks the ingot is made thinner to form transverse hinges 25 which facilitate bending of the ingot in the plane thereof. For the manufacture of the brushes these are to be advanced along a process line along which there are provided several working stations, and this transport takes place with the brush shanks integral with each other in the manner shown in FIG 12. A metal profile 26 of the kind shown in FIG 13 can be used for guiding the integral shank set along the line. This profile has a downwards open undercut groove 27 dimensioned to receive therein the ingot 24 and to support same, and it has also at the sides thereof undercut grooves 28 for mounting the profile in a frame or the like.

For advancement of the shanks there can be provided in the profile 26 apertures at the sides thereof for engagement of driven clamp rollers with the sides of the ingot, or another drive means can be provided, such means being easily proposed by the skilled man.

When the shank set of FIG 12 has been removed from the injection molding machine it is inserted manually into the guide formed by the profile 26 then to be taken over by the drive means and be advanced along the process line. In order that the surface of the shanks shall be made optimally adherent the shanks are pretreated at 10-20 kV and 2-3 A then to be coated with glue, preferably a water-based glue, over a predetermined length at the brush end of the shank, i.e. over the end portion of the shank which is to be provided with fibers. This can be made according to FIG 14. The line formed by the profile 26 has a downward bow 29 so that the shanks 15 are lowered and also are mutually dispersed, and below said downward bow there is a trough 30 with glue. In the glue trough a predetermined level is maintained by supplying glue from a supply vessel 31 and returning glue to said vessel via an overflow 32. Thus, an accurately defined end portion of the shank will be provided with glue when the shank during the movement thereof along the process line is dipped into the glue trough. The glue can be replaced by a solvent for the shank material so that the fibers are attached in a dissolved sticky surface layer of the shank.

In a following station shown in FIG 15 application of fibers on the end portion of the shank provided with glue, then is effected by electrostatic flocculation. Fibers such as nylon fibers of a predetermined uniform length are received in a trough 33 connected to the positive pole of an AC source the voltage of which is e.g. 90 kV, and the fibers are supplied to the trough and are kept loose therein by means of a feeder screw 34. Also over the trough 33 the profile 26 forms a downward bow 35 so that the shanks will be moved downwards towards the trough and at the same time will be dispersed when they pass along the process line. The profile is grounded, i.e. it is connected to the negative pole of the high voltage source, and by known technique the fibers are drawn to the shanks where they are "shot" into the portion coated with glue and will be attached to said portion. As a consequence thereof they will form a velour finished portion over that part of the shank which is coated with glue, and in this portion the fibers stand on end projecting radially from the shank. The density of the velour finished portion which thus forms the brush proper will be dependent on the dwelling time of the shank in the electrostatic field and of the strength thereof. After velour finishing the brushes are cut from the ingot and are enclosed in sterile packages.

## Claims

1. Brush comprising a shank which is provided with bristles over a portion of the length thereof, preferably over an end portion, **characterized** in that the portion (11) provided with bristles comprises a velour finished surface having fibers which are attached to the shank and stand on end projecting from the surface of the shank.

2. Brush as in claim 1 wherein the velour finished surface of the shank is shaped to conform with a sampling place.

3. Brush as in claim 2 wherein the velour finished surface shaped to conform with the sampling place comprises a separate body connected with the rest of the shank.

4. Brush as in claim 2 wherein the velour finished surface is provided on a conical body having the small end at the end of the shank.

5. Brush as in claim 4 wherein the conical body has two or more flaps which can be folded towards the shank.

6. Brush as in claim 1 wherein the velour finished surface is provided on a circular disk the shank being connected centrally and axially to the disk.

7. Brush as in claim 1 wherein the velour finished surface comprises several axially mutually spaced annular portions.

8. Brush as in claim 1 wherein the velour finished surface comprises a helical string.

9. Brush as in claim 1 wherein the velour finished surface comprises several radially mutually spaced axial portions.

10. Brush as in claim 1 wherein the velour finished surface is provided on the end portion of a spatula.

11. Method in manufacturing a brush comprising a shank having an end portion provided with bristles, which comprises a velour finished portion with fibers which are attached to the shank and stand on end projecting from the surface thereof, **characterized** in that the end portion is dipped in glue or solvent for the shank material and that the fibers then are applied by being adhered to the shank by means of an electrostatic field so as to be attached to the end portion coated with glue or partly dissolved.

## Patentansprüche

1. Bürste, mit einem Stiel, der an einem Abschnitt seiner Länge, vorzugsweise an seinem Endabschnitt, mit Borsten versehen ist, dadurch **ge-**

**kennzeichnet**, daß der mit Borsten versehene Abschnitt (11) eine Velourappretur-Oberfläche mit Fasern aufweist, die an dem Stiel befestigt sind und auf einem Ende stehen, wobei sie von der Stieloberfläche abstehen.

2. Bürste nach Anspruch 1, bei der die Velourappretur-Oberfläche des Stiels so geformt ist, daß sie mit einer Probenentnahmestelle übereinstimmt.

3. Bürste nach Anspruch 2, bei der die Velourappretur-Oberfläche, die in Übereinstimmung mit der Probenentnahmestelle geformt ist, einen getrennten Körper aufweist, der mit dem Rest des Stiels verbunden ist.

4. Bürste nach Anspruch 2, bei dem die Velourappretur-Oberfläche an einem konischen Körper vorgesehen ist, der das schmale Ende am Ende des Stiels hat.

5. Bürste nach Anspruch 4, bei der der konische Körper zwei oder mehr Flügel besitzt, die in Richtung auf den Stiel gefaltet werden können.

6. Bürste nach Anspruch 1, bei der die Velourappretur-Oberfläche mit einer kreisförmigen Scheibe versehen ist, mit der der Stiel mittig und axial verbunden ist.

7. Bürste nach Anspruch 1, bei der die Velourappretur-Oberfläche mehrere axial voneinander beabstandete ringförmige Abschnitte aufweist.

8. Bürste nach Anspruch 1, bei der die Velourappretur-Oberfläche eine schraubenförmige Kette aufweist.

9. Bürste nach Anspruch 1, bei der die Velourappretur-Oberfläche mehrere radial beabstandete Axialabschnitte aufweist.

10. Bürste nach Anspruch 1, bei der die Velourappretur-Oberfläche an dem Endabschnitt eines Spatels vorgesehen ist.

11. Verfahren zum Herstellen einer Bürste, die einen Stiel aufweist, der einen mit Borsten versehenen Endabschnitt aufweist, der einen Velourappretur-Abschnitt mit Fasern aufweist, die an dem Stiel befestigt sind und abstehend von seiner Oberfläche auf einem Ende stehen, dadurch **gekennzeichnet**, daß der Endabschnitt in Klebstoff oder ein Lösungsmittel für das Stielmaterial eingetaucht wird und daß die Fasern dann durch Haftenbleiben an dem Stiel mit Hilfe eines elektrostatischen Feldes aufgebracht werden, so daß sie an dem mit Klebstoff überzogenen oder teilweise gelösten Endabschnitt haftenbleiben.

**Revendications**

1. Brosse comprenant une tige qui comporte des soies sur une partie de sa longueur, de préférence sur une partie d'extrémité, caractérisée en ce que la partie (11) comportant des soies présente une surface au finissage velours ayant des fibres qui sont fixées à la tige et sont dressées sur un bout en étant en saillie sur la surface de la tige.

2. Brosse selon la revendication 1, dans laquelle la surface au finissage velours de la tige est façonnée de manière à épouser la forme de l'endroit où il y a prélèvement d'échantillons.

3. Brosse selon la revendication 2, dans laquelle la surface au finissage velours façonnée de manière à épouser la forme de l'endroit du prélèvement d'échantillons comprend un corps séparé relié au reste de la tige.

4. Brosse selon la revendication 2, dans laquelle la surface au finissage velours est prévue sur un corps conique ayant sa petite extrémité au bout de la tige.

5. Brosse selon la revendication 4, dans laquelle le corps conique comporte deux pattes ou plus qui peuvent être pliées vers la tige.

6. Brosse selon la revendication 1, dans laquelle la surface au finissage velours est prévue sur un disque circulaire, la tige étant reliée au centre du disque et suivant l'axe de celui-ci.

7. Brosse selon la revendication 1, dans laquelle la surface au finissage velours comprend plusieurs parties annulaires espacées axialement les unes des autres.

8. Brosse selon la revendication 1, dans laquelle la surface au finissage velours comprend un cordon hélicoïdal.

9. Brosse selon la revendication 1, dans laquelle la surface au finissage velours comprend plusieurs parties axiales espacées radialement les unes des autres.

10. Brosse selon la revendication 1, dans laquelle la surface au finissage velours est prévue sur la partie d'extrémité d'une spatule.

11. Procédé de fabrication d'une brosse comprenant une tige ayant une partie d'extrémité munie de soies, qui comprend une partie au finissage velours avec des fibres fixées à la tige et dressées sur leur bout en étant en saillie sur la surface de la tige, caractérisé en ce que la partie d'extrémité est plongée dans une colle ou un solvant pour la matière de la tige et en ce que des fibres sont alors appliquées en étant amenées à adhérer à la tige au moyen d'un champ électrostatique de manière à être fixées à la partie d'extrémité revêtue de colle ou à être partiellement dissoutes.

FIG. 1

FIG. 2

FIG. 3

FIG. 4

FIG. 5

18

16

15

15'

FIG. 6

19

15

15'

FIG. 7

20

FIG. 8

FIG. 9

FIG. 10

FIG. 11

FIG. 12

FIG. 13

FIG. 14

FIG. 15